Europäisches Patentamt

European Patent Office

Office européen des brevets

(11) Publication number : **0 469 227 A1**

# (12) EUROPEAN PATENT APPLICATION

(21) Application number : **90830570.9**

(22) Date of filing : **07.12.90**

(51) Int. Cl.⁵ : **A61M 21/00**

(30) Priority : **31.07.90 IT 4818290**

(43) Date of publication of application :
**05.02.92 Bulletin 92/06**

(84) Designated Contracting States :
**DE FR GB IT NL**

(71) Applicant : **EUROLOG S.R.L.**
**Via Arminjon 5**
**Roma (IT)**

(72) Inventor : **Lancia, Ezio Maria**
**Via Cassia, 929**
**I-00189 Rome (IT)**

(54) **Electronic device controlling light sound and magnetic field levels to induce sleep or to condition circadian rhythms.**

(57) A device suitable to control three different sources of energy : sound (music level), light (brightness) and magnetic field, capable of programming, by the use of a plug in clock, the timing of the energy emissions and to establish their respective levels, equally provided with a sensing device, such a "baby crying" transducer, in such a way as to induce the user of the device into a condition of sleep or to bring back to the original timing an otherwise disturbed circardian rhythm, as usually experienced in the "Jet lag" syndrome.

The device can be manually controlled or can use a macroprocessor.

EP 0 469 227 A1

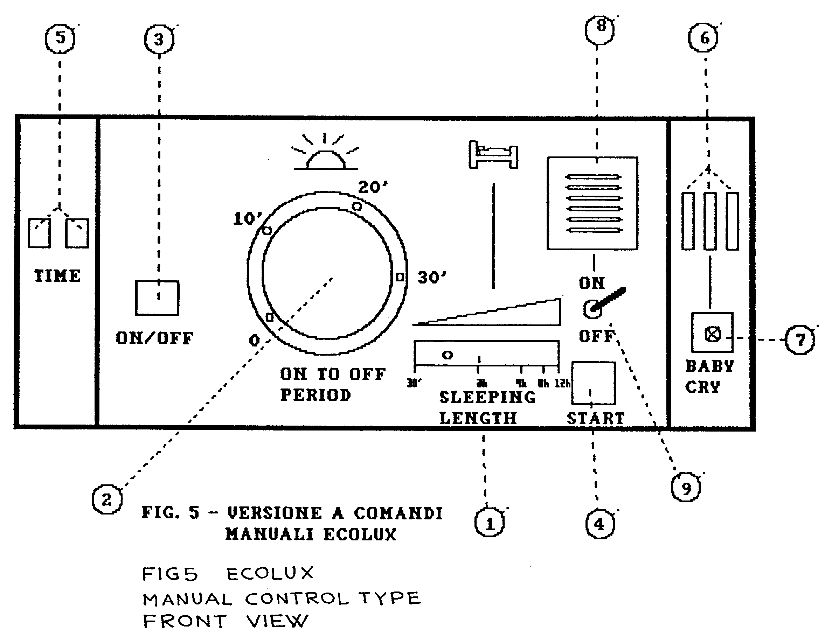

FIG. 5 - VERSIONE A COMANDI
MANUALI ECOLUX

FIG5 ECOLUX
MANUAL CONTROL TYPE
FRONT VIEW

## 2.I. ANALYTICAL DESCRIPTION OF THE DEVICE.

### 2.1.1. What is "ECOLUX"

"ECOLUX" is an electronic device which allows to automatically adjust three main parameters: light, sound and magnetic field, in order to give a contribute in solving sleep problems of any age persons.

Such parameter are combined in order to help the sleep, either to produce it, or to keep it, or to adjust it.

"ECOLUX" represents the results of studies and researches, made by means of specific experiments and applications, speeded up by the questions concerning sleep mechanisms (refer for such purpose to existent bibliography, and to the considerations about sleep's nature and the relative troubles, described at points c) and d) of this paragraph).

"ECOLUX" allows to adjust the light intensity according to fig. I (variation of the luminous intensity in function of time): the light, during the turning - off (phase b) is automatically adjusted according to a law similar to a sunset. Such phase may be accompanied by a sweet and low music.

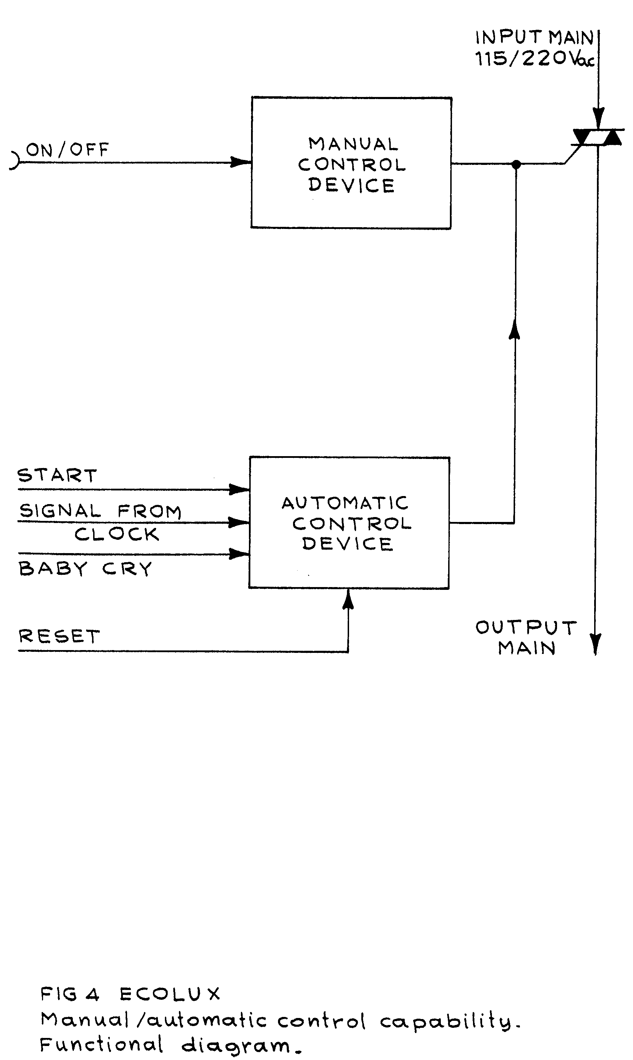

The main principle, from which the idea of this device was born, is derived from the fact that a person sleeps better when he is quiet, therefore it is of a fundamental importance the "safety" factor, especially for children, often troubled by night - terror.Being well - known that a child, waking up during night, is afraid of darkness, a circuit available on a "plug - in" module, BABY CRY ALERT, is conceived, in order to detect child cries,obtaining automatic light-on: the light is again automatically lighted - off after some minutes since the child's cries are ended; a pleasant sound, besides, is given out (fig.4).

"ECOLUX" is conceived in such way that persons is not "abandoned" during the sleeping period, therefore the three fundamental variables are automatically adjusted and combined in a right manner:

I) Light: a variable luminosity, at low intensity, is present

2) Music: it is sweet and low;

3) A little variable magnetic field, having very low frequency, is generated.

The interaction between the low intensity light and the open eyes during REM contributes to keep a certain muscular tone, in order to attenuate snoring too.

In CPU automatic model, the three main parameters' variations may be stored and custom - made in relation to a specific sleep brain wave diagram. From actual studies, the interaction between magnetic field at low temperatures frequency (6Hz) generated by "ECOLUX" and the magnetic waves generated at the same frequency by the electrical signals produced by the human brain during the sleep stages, helps maintaining the sleep itself.

By means of this device, the following parameters may be selected:

− the lighting - off preferred curve;

− the sleeping period;

− the sleep's parameters' variations according to individual needs

"ECOLUX" may be useful for:

− a married couple, because it tries to make sleep's modes uniform.

− women in advanced pregnancy state because baby accustoms itself, in the before birth period, to follow certain rhythms of sleep.

### 2.1.2. MODELS / USERS (Table I)

### MAIN CHARACTERISTICS (Table 2)

In the annexed tables the main characteristics of this device family are summarized.

For all devices either the on wall type or the portable type exist (Table I).

All types are automatic and are available:

I) with manual controls

2) with digital controls, without CPU

3) with digital controls, CPU model.

The CPU model keeps into its memory a luminous curve variable in function of sleep's stages. Such curve may be programmed in function of individual needs.

They are universal devices: any ac voltage may be applied, any lamp with any base may be matched. They are furnished with universal plugs and sockets for any connection type.

As an option, a "plug - in " clock module may be supplied, in order to allow an automatic programming of sleep's starting instant during the every day's 24 hours. The "safety" factor is ensured, during the phase of falling asleep, by the presence of an automatic light variation ( from "on" to "off", similar to a sunset ),accompanied by a low and sweet sound.

## 2.1.3.SLEEP; EFFECTS OF EXTERNAL STIMULUS (LIGHT, SOUND)

Note: some information, here reported, are derived from EUROPEAN ENCYCLOPEDIA - Ed. Garzanti.

It is irrefutable that no man can do without sleep even if its duration changes for each person.

It is important to do some considerations about constitution and nature of sleep, in order to better correlate among sleeping parameters and the usefulness and the characteristics of the devices object of this specification.

For what concerns sleeping constitution, the brain wave diagram (hystogram) of each person can be detected applying electrodes to the scalp, to the orbits and to the chin muscles.

The sleep is preceded by a waking stage E and may be subdivided in five fundamental stages.

In adults, the different sleeping stages follow in time order according to their depth, valuable with the intensity of one stimulus, capable to provoke the reawakening.

As it results fron experiments, dont by us, a "natural" stimulus is the light itself, which united to an eventual sweet and low sound, can influence upon the sleeping depth, in function of modulation or oscillation of the luminous intensity in relation to some fixed reference values.

The "initial stage" (E) is relative to the passage from consciousness to sleepiness, that is the prelude to the true sleep.

The stage I is the first stage of drowse accompanied by an increase of electrical cerebral activity and by a decrease of muscular tone.

The stages II, III, and IV are characterized by a sleeping increasing depth and by a slackening of electrical cerebral intensity and by the presence of a certain muscular tone level.

Finally, the last stage, the fifth, is the REM which always comes after a preparatory slow sleep period of about ninety minutes and is deep as that of III and IV stages: its peculiarity is constituted by a rapid electrical activity, by rapid eyes movement (REM) and by complete absence of muscular tone.

The REM would correspond to a periodical genetic reprogramming of brain, responsible of keeping the psychological inheritance. It constitutes the "inheritable part of typology of each man" (genetic revival).

Sleeping period is subdivided in cycles: every cycle is made up by a slow sleep phase and by a "sound"sleep phase.

The "sound"sleep REM lasts 15 - 20 minutes, while a complete cycle lasts 100 minutes.

A normal eight hour sleep is made up by five cycles; the duration and number of cycles changes according to the type of man and for each man according to his age.

For this reason, as device control fundamental parameter, cycles' number and their duration also is utilized.

## 2.1.4 SLEEP PROBLEMS AND TROUBLES

The stimulus produced by "ECOLUX" devices may give a contribute to the solutions of some pathological alterations connected to sleep, as the hypersomnia,which may be due to a deficiency of activator mechanisms of awakening system, or as the sleeplessness, otherwise defined as the subjective sensation of non sleeping, or as the drowsiness, or as the snoring.

The snoring gives evidence to the difficulty of air passage through the superior aerial ways because of a minor muscular tone, and of oscillatory movements of the tongue backwards.

In some cases it may cause to stop the respiration, that is of obstructive origin, but also of central origin. Even the sudden death may be caused (specially in suckling).

The trouble of snoring is present specially in the "REM" of which phase is characterized by the rapid movement of the eyes, by the dreaming and by the absence of muscular tone.

Increasing the luminous intensity in relation to a reference low level, a decrease of sleeping depth may be caused during the "REM" in order to obtain an increase of muscular tone, such as to lessen the snoring phenomen.

Another problem that a good sleep contributes to its resolution is the regular growth during children development; in the sleep's stages III and IV the hormone of growth is secreted. As a matter of fact it has been possible to attribute some delays of the growth to the bad sleep due to uncomfortable economical / sanitary condition (noise, promiscuity) because growth's problems are solved well only if the interested child is under conditions to sleep correctly.

For what concerns the hypersomnia, the sleeplessness, the drowsiness, these troubles are essentially due to an alteration of the "biological clock" of which we explain meaning

BIOLOGICAL CLOCK AND SLEEP

The alteration of waking and sleeping, depends, for the greatest part of the species, upon the following of night and day, which acts as synchronizer or time pointer in relation to a biological clock placed in the particular brain's region, called "hypothalamous".

This internal clock controls many other watches that direct the enzymatic and hormonal synthesis, the body's temperature and, in an indirect way, the sleeping.

Industrial civilization, with present irregular life's rhythms (works divided into turns, journeys from one continent to another, journeys done generally in night hours, summer - time) has put the human brain in conditions not foreseen by evolution.

For example, a traveller who leaves Milan for Los Angeles preserves the endogenous, about daily, rhythm of his internal clock, but receives sensorial information of night and day, shifted by eight or nine hours, causing an asynchronism between his sleeping need and local time.

If may be understood therefore how much it is important to look after the possible asynchronisms induced by external agents upon the sleeping waking cycle present in every body.

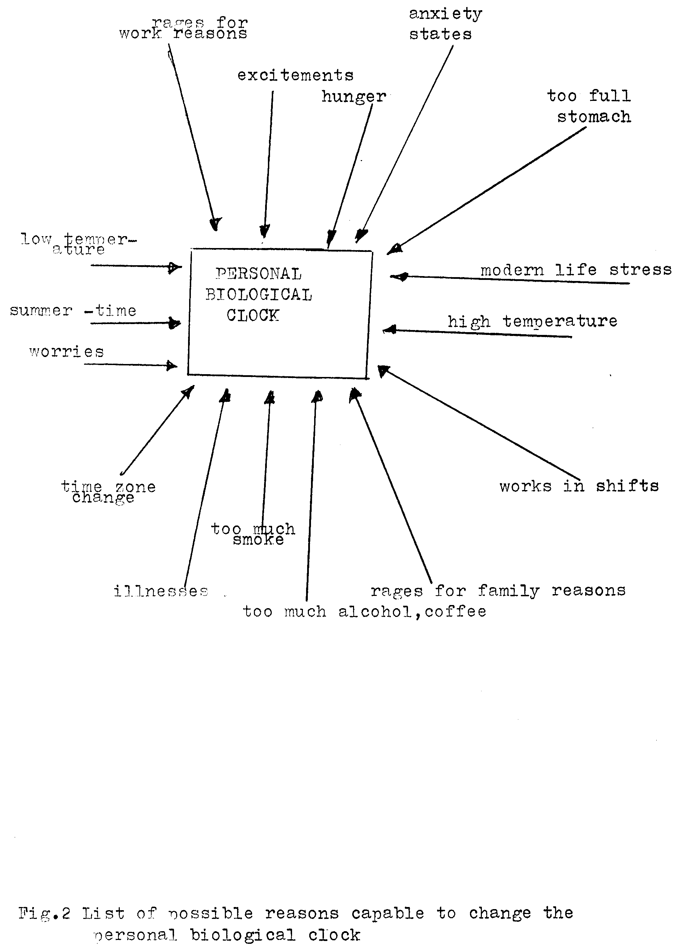

For example, in fig.2, the possible reasons that may change the biological clock, are put in evidence.

Every man needs to dedicate a specific time to sleeping.

By the doctor's assistance, selecting the cycles' number and their duration, synchronization of the out of phase internal biological clock may be favoured.

Therefore, the doctor can custom - make the use of ECOLUX to the type of patient, after detecting the brain wave diagram(hystogram) of a sleeping night, fixing electrodes to the scalp, to the orbits and to the chin muscles.

## Par. 2.2. PRESENT STATE OF TECHNICS

At the present similar and /or having similar purposes devices don't exist.

As far as it is known, on the market devices, only capable to control manually the luminous intensity, exist.

These devices have generally the function to adjust pleasant /preferred diffused lighting in an ambiant, at a certain instant.

Besides, on the market, device with various functions are available.

Some functions are for example:

– Light timing switches (on/off)

– Sensors that control the turning - on of heating and or irrigation system, and so on, in relation to luminous intensity (for example at the sunset), that are nothing to do with the here described invention.

## Par. 2.3. DESCRIPTION OF THE DEVICE

### 2.3.1. STANDARDS, INSTALLATION, COMPOSITION.

This device is done in two main types

I) One wall embedment type

2) Plugged / portable type and / or type for bedside installation.

The difference between the two types consists mainly in the outer interface: namely, the on wall type is the model that shall be installed in place of pre - existing switches of which the external mechanical structures, the switch function and the outer interface (made up by light wires) are preserved.

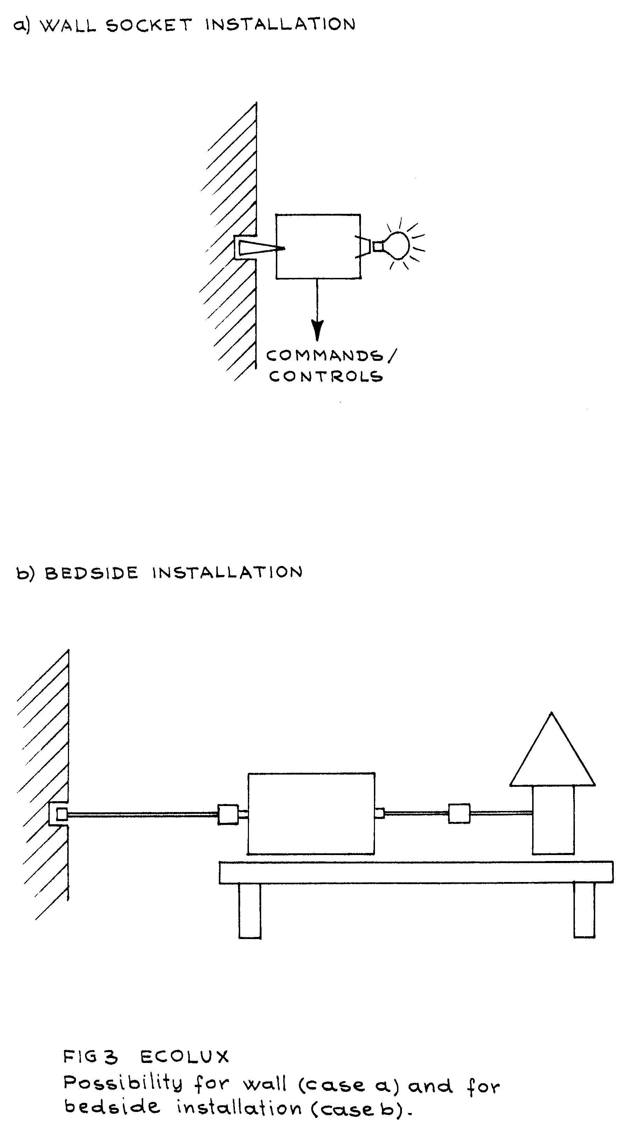

Plugged / portable and / or for bedside installation type, though it keeps the same operative capabilities and therefore the relative commands and controls, has the interfaces to be able to satisfy the installation needs shown in fig.3: it will be furnished therefore with an universal socket (Vimar), adaptable to any type of plug, with a standard plug, with an extension cable for connection to the wall and of type suitable to the pre - existing socket as accessory, with a socket for lamps having great base: with suitable reducers, different wall - socket or different lamps for example with mignon base, can be matched.

The composition of "ECOLUX" devices is the following:

I) Manual control type:

   – Case

   – Power Supply board

   – Magnetic field generator board

   – Actuators and phase detector board

   – Plug - in "BABY CRY ALERT" Submodule

– Plug - in CLOCK submodule

– N. I plug reducer

– N. I extension cable, supplied with two connectors: one standard plug and one universal Vimar socket.

– N. I reducer for lamps with mignon base.

2) Digital Control model

It has the same composition as the previous type with the addition of:

– N. I Display LCD control board

The logic control board will be different from the previous, being without the analogic to digital conversion and having the necessary interface circuits for the external commands.

3) Digital Control model, programmable by means of CPU.

This will have in relation to the previous type :

– N. I CPU board, in place of the control logic board.


2.3.2. OPERATION (COMMANDS AND CONTROLS OF "ECOLUX" DEVICES)

These devices will have all the necessary commands to set automatic lighting - on times (sleeping duration ) and gradual lighting - off times.

Besides, being three the controlled parameters (light, magnetic field, and music), ECOLUX has also the control circuits for magnetic field and sound level generation.

In fig. 4 a functional diagram is shown indicating the possibility to use it as a simple switch, releasing from automatic control: it is enough to act on on /off touch control pushbutton to be able to turn - on or to turn - off the light, with a sharp blow, or manually to change the luminous intensity gradually, by means of a simple continous pressure.

Simply acting on START pushbutton automatic control is activated, while, acting on on /off pushbutton, manual control is activated.

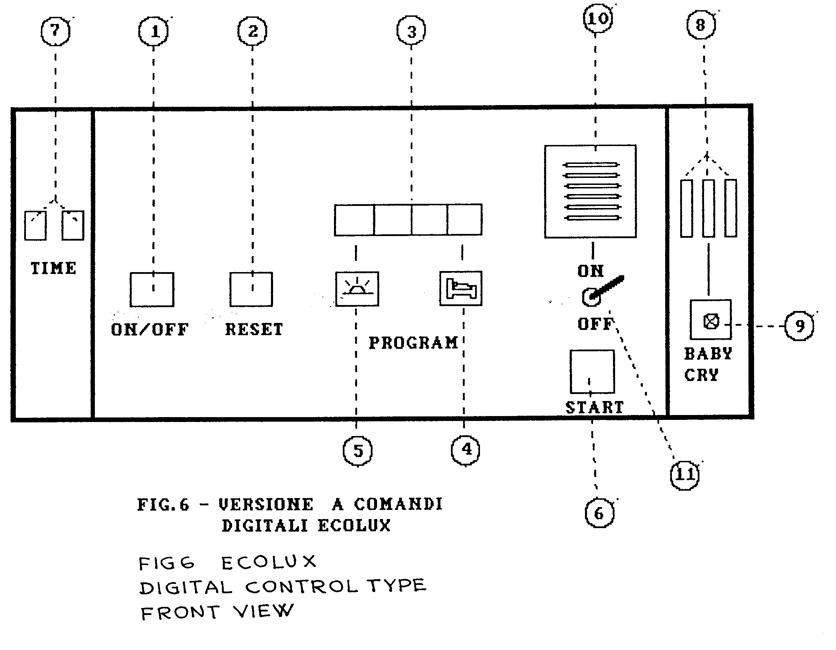

This possibility, above described, is available on all types, as it can be deduced by layouts of fig.5 and 6.


2.3.2.1.LAYOUT DESCRIPTION OF "ECOLUX" MANUAL CONTROL MODEL

In fig. 5 the layout representing commands / controls disposition of manual control type is shown.

The meaning of the commands / controls is the following:

I) it is used for adjusting the duration of period having about zero luminous intensity. This duration is variable form I/2 hour to twelve hours.

2) It is used for changing the necessary time to pass from full lighting - on phase to lighting - off phase (on to off period).

3) on / off touch control pushbutton;

4) START pushbutton: if pressed, luminosity control cycle starts.

5) "Plug - in " Clock: it is used for starting the luminosity cycle at a fixed time, during the 24 hours of every day

6) Detector of Children' cries.

7) Activation of baby cry detector.

8) Loudspeaker, for the diffusion of a sweet and low music during the lighting off phase and during sleeping lenght.

9) Switch for music application to the loudspeaker.


2.3.2.2. LAYOUT DESCRIPTION OF THE DIGITAL CONTROL MODELS

In fig. 6 layout of "telemathic" type is shown.

The meaning of commands / control is:

I) on /off touch control pushbutton

2) RESET pushbutton: it is used for resetting programmed times.

3) liquid crystal display (LCD) : for visualizing programmed times.

4) Pushbutton which is used for programming sleeping period duration.

5) Pushbutton which is used for programming necessary time to pass from full lighting - on phase to lighting - off phase ( on to off period).

6) START pushbutton: if pressed, luminosity control cycle starts.

7) "Plug in" clock (option)

8)9) "Plug in" - Baby Cry Alert (option)

10) Loudspeaker for diffusion of a sweet and a low music during the lighting - off phase and during sleeping lenght.

II) Switch for music application to the loudspeaker.

## 2.3.3. TECHNICAL DESCRIPTION.

### 2.3.3.1. GENERAL FUNCTIONAL DIAGRAM

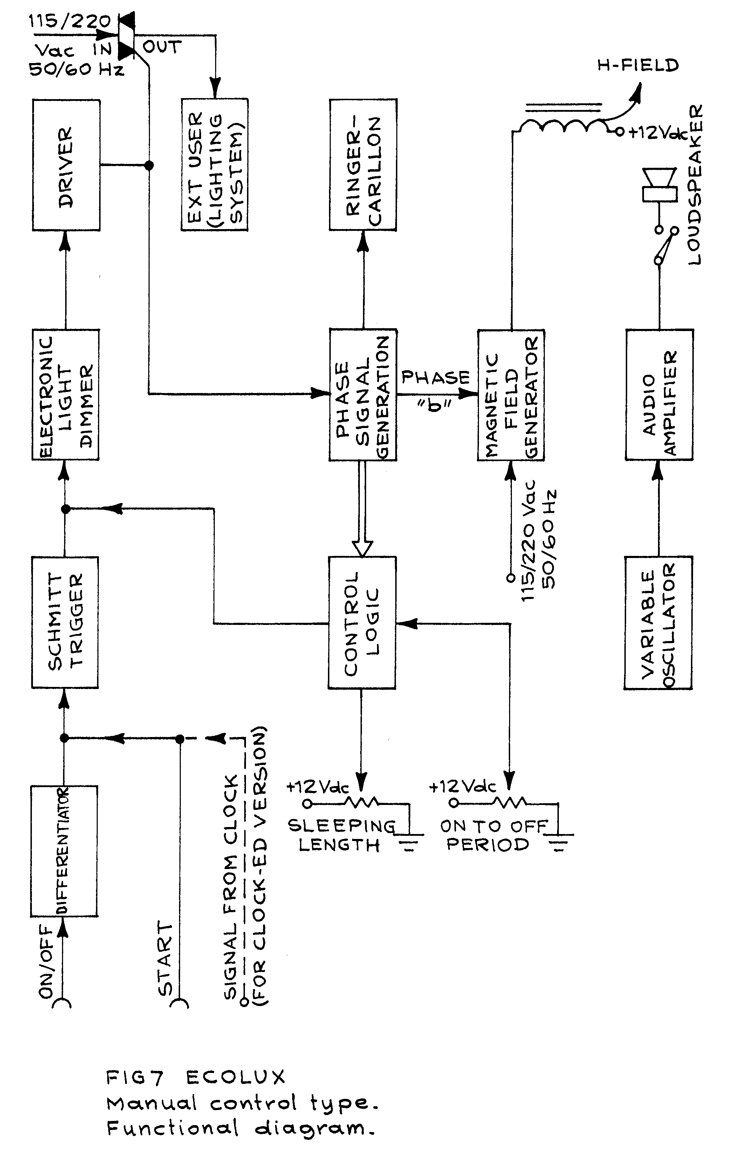

In Fig. 7 a general functional diagram of ECOLUX device is shown.

Impulses, generated by touch control ON/OFF pushbutton, are differentiated, triggered. By means of dimmer integrated circuit and the relative driver, the output triac is controlled in order to adjust the alternate voltage for the light system.

By means of two variable resistors, "on" to "off" period and sleeping lenght durations can be adjusted.

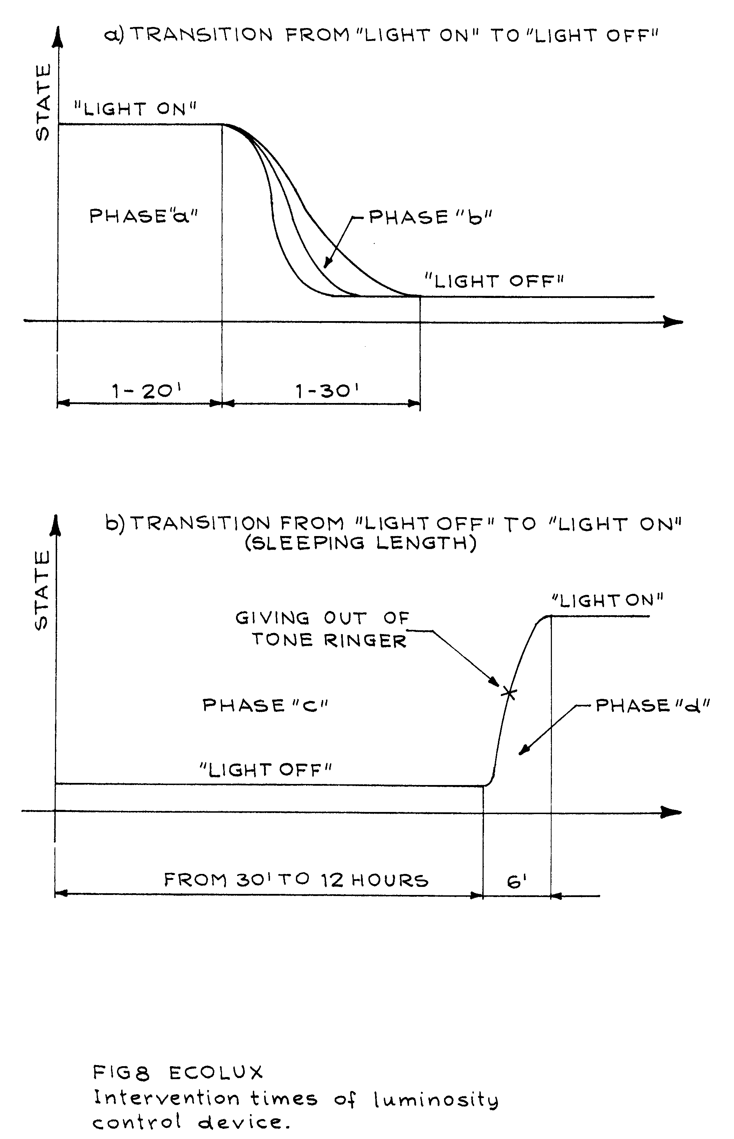

In the fig. 8 the characteristics times of luminous intensity duration are represented.

Notice furthermore that during the light - off period the luminous intensity is not reduced to zero: a dim luminous intensity is present; so who wakes up during night, doesn't see complete darkness, and gets a sense of greater safety.

These curves of luminosity variation are obtained by means of control logic circuits and by means of S 566 A Electronic Light Dimmer integrated circuits and relative driver:in the following these circuits are described.

A circuit generates the signals, relative to every phase, while a window comparator activated during the phase "d" (awakening), allows to activate a ringer like a "carillon".

The presence of a variable oscillator enables to apply, by means of an amplifier, a pleasant and low music to a loudspeaker, in order to transmit a sence of quietness and safety during the falling asleep phase and the true sleeping period.

Another parameter that favours the sleep is the magnetic field variable with the same freqency as of the electrical impulses generated by human brain.

Magnetic field intensity will be of a quite greater magnitude (about 5 + 10 gauss) as terrestrial one (0.5 gauss).

The START pushbutton has the function to activate the mechanism of variable luminous intensity: such mechanism can be operated also by the signal coming from the clock, in order to activate it at a determined hour in the twenty - four hours of each day (clock model)

### 2.3.3.2. CONTROL LOGIC

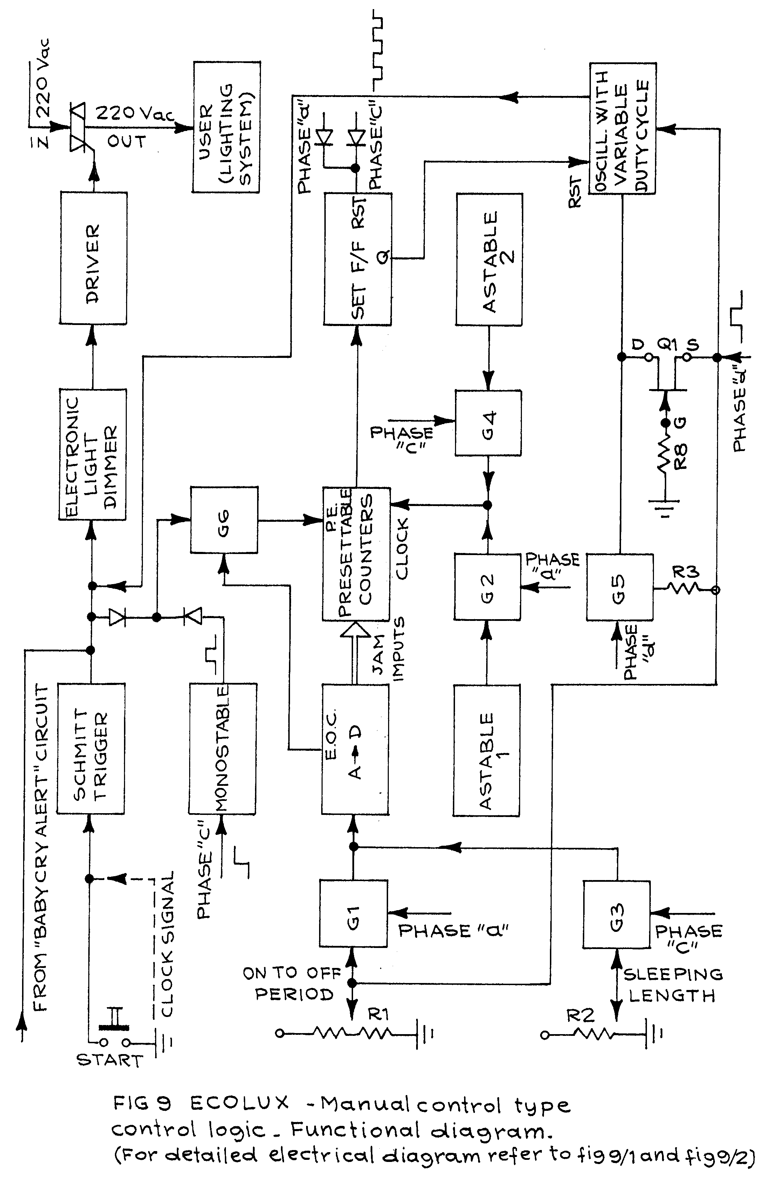

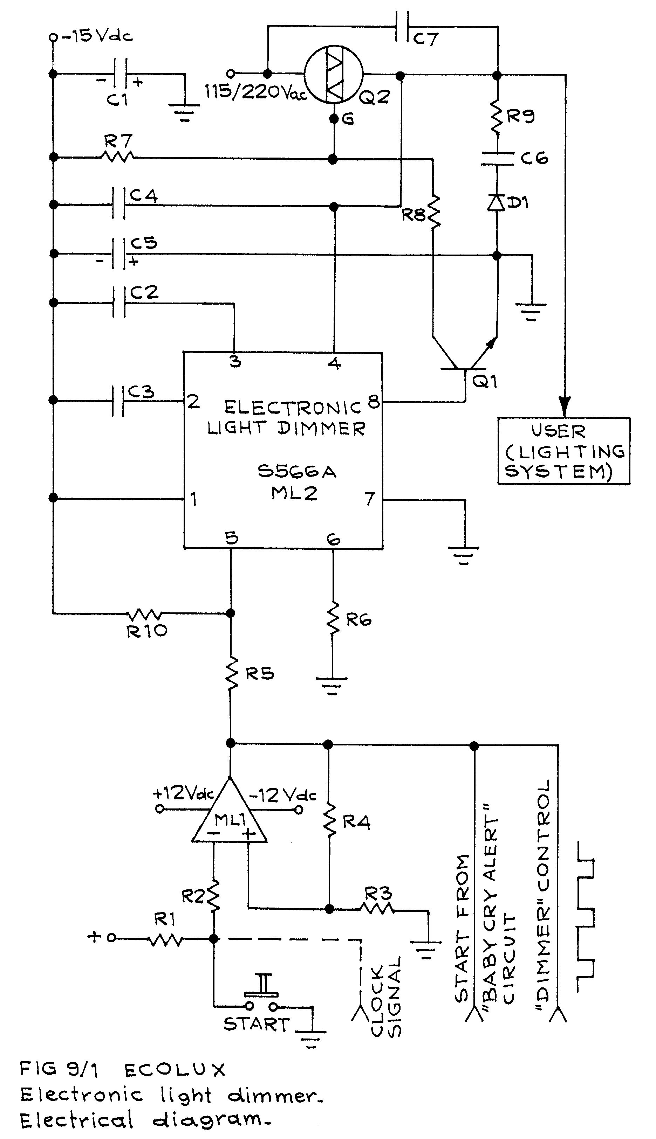

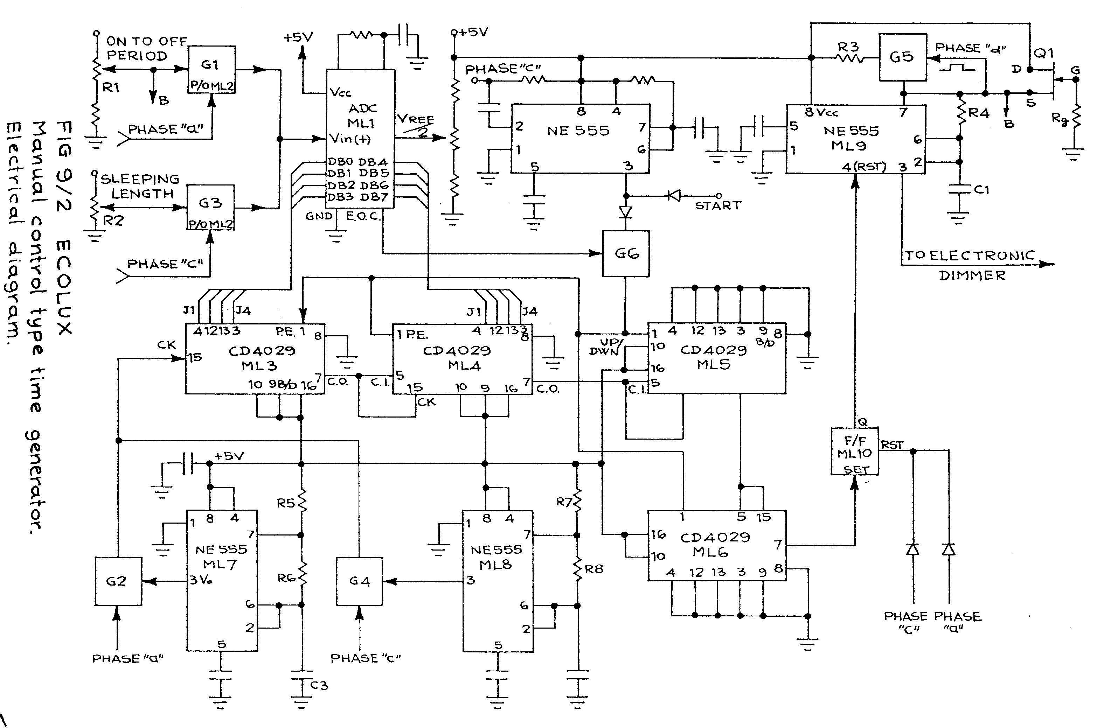

In Fig. 9 control logic functional diagram is shown.

The output level from the variable resistor (RI), ON TO OFF PERIOD, is applied during phase "a" by means of GI (GI, solid state switch), to analogic to digital converter.

The converter's digital outputs preset the counter, which after a time proportional to phase "a" duration, activates one oscillator having a variable "duty cycle".

The period of this oscillator is adjusted by means of the variable resistance, obtained by suitable biasign Q I (VCR) with the voltage coming from the variable resistor (RI).

The oscillator's period provides to change the phase "b" duration (period during which the light is turning to off).

Similarly, output level of variable resistor R 2, SLEEPING LENGHT is applied, by means of G 3, to analogic to digital converter, in the phase "c" (sleeping period), in order to preset the counters with codes corresponding to the sleeping lenght duration.

The oscillator with variable "duty cycle" has a period determined by the R 3 resistor, switched - in during the phase "d" (period during which the light is turning - on) by means of switch G 5.; the phase "d" duration is constant and equal to six minutes.

The preset counters are driven by two astable oscillators, of which the one at low frequency is applied by means of G4 during the phase "c" (longer durations) and the one at high frequency by means of G2 during the phase "a".

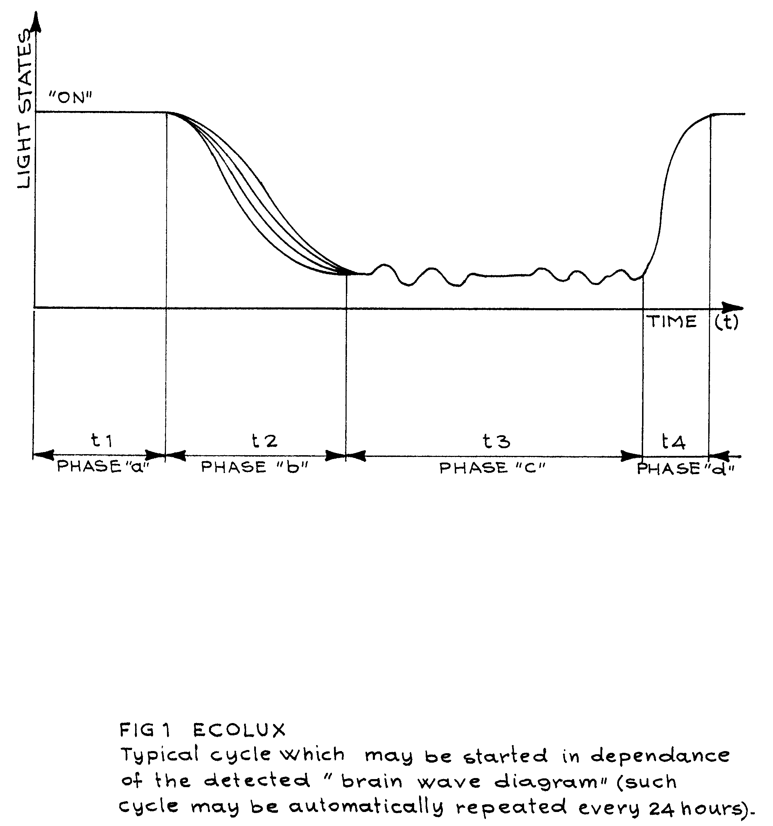

The counters are preset at the beginnings of the phases "a" and "c" when the analogic to digital converter has generated a signal of "end of conversion" "EOC". In fig. 9/1 a detailed electrical drawing relative to the Dimmer actuator is shown.

The fig. 9/2 represents a detailed electrical drawing relative to the circuits which generate the intervention times of luminosity control.

### 2.3.3.3. PHASE SIGNAL GENERATION CIRCUITS.

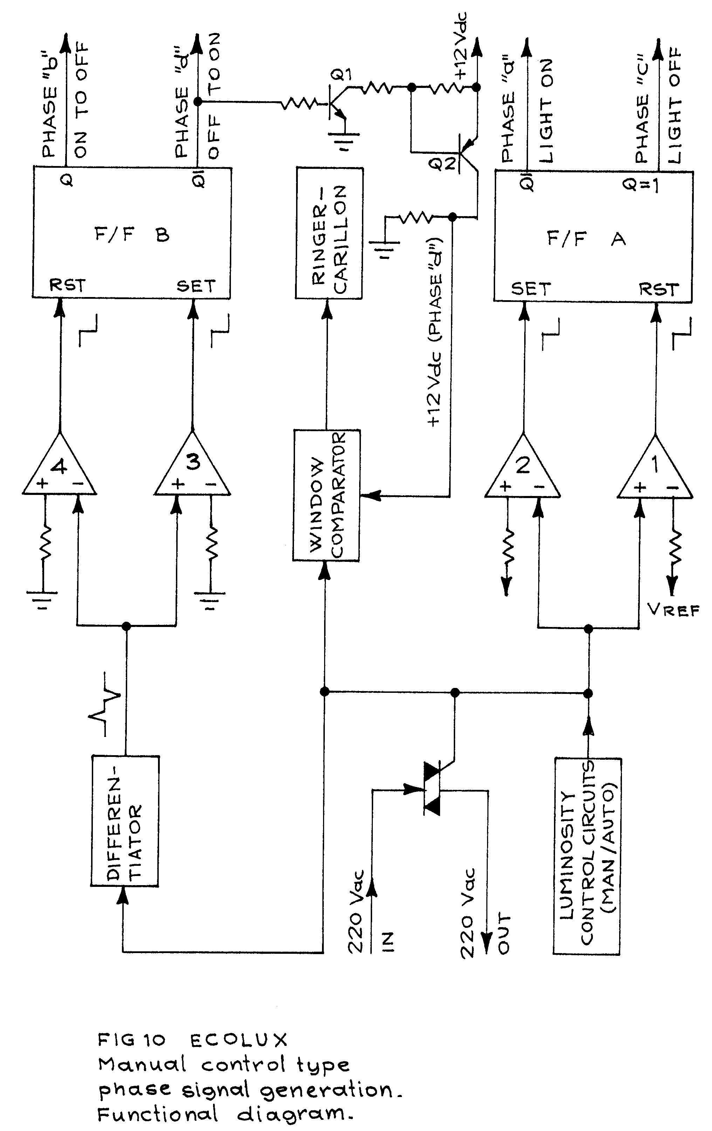

In fig. 10 a functional diagram concerning the circuit of various temporal pahse generation is shown.

The signal applied to the triac, necessary to obtain the main voltage variation, is compared by means of comparators 1 and 2.

The comparator I output becomes high when the light is "on" and F/FA flip flop is reset by comparator I output pulse.

Therefore $\overline{Q} = I$, such signal is the phase "a" signal.

When the light is about "off" comparator 2 output becomes high and sets F/FA. So Q=1, and this signal is the phase "c" signal.

A circuit that performs the derivative provides to generate positive or negative pulses, relative respectively to phase "b" or "d".

Comparator 3 output becomes high during the phase "b" and F/FB is set. F/FB Q output becomes high: therefore this Q is the signal relative to phase "b".

Comparator 4 output becomes high when the output pulse of differentiatior circuit is negative, the F/FB is reset, manely $\overline{Q} = 1$, phase "d" signal.

This phase "d" signal provides by means of QI and Q2 transistors to generate the + 12V (phase "d") for the window comparator which makes ringer operate during the phase "d" (awakening period).

### 2.3.3.4. MAGNETIC FIELD GENERATION.

As it is demonstrated, the slep is favoured orienting own body according to the external existing magnetic field such as to rightly interact with magnetic field generated by cerebral waves;therefore an adjustable magnetic field is created.

The main voltage 220 Vac, 50 Hz, is attenuated, squared and suitably divided.

I) Normally ac voltage is divided only by eight, getting a frequency equal to about 6 Hz, that is the one more usual of cerebral electrical waves.

2) In CPU model, dividing the 50 Hz frequency by 3, by 8 and by 12 frequencies of about 16 Hz, 6 Hz and 4 Hz respectively are got: these frequencies are switched according to CPU logic in function of the parameters taken from the measured and stored brain wave diagram.

The signal, having these frequencies, is amplified by darlington Q1 and Q2.

The inductance L, connected to darlington's collector, provides to generate the relative magnetic field.

The inductance L is made up by two C cores, FA 35 Q13; being the 6 Hz fundamental voltage at the ends of the inductance equal to 10.7 Vrms, it will be got:

$10.7 = 4.44 \, f \, \Phi \, N.$ from which supposing $\Phi = B . Sfe$

$B = 5 \text{ web} / m^2 \; Sfe = 0.95 \text{ cm}^2, f = 6 \text{ Hz}$

$\Phi = 0.95 . 10^{-4} . 5 \text{ web} = 4.75 . 10^{-4} \text{web}$

therefore :

$$N = \frac{10.7}{4.44 . 6 . 4.75 . 10^{-4}} = 845 \text{ turns}$$

The inductance value will be

$L = \dfrac{N^2}{R_a + R_{iron}}$ and being $\mu = 1.375 . 10^{-2}$

$R_{fe} = \dfrac{1}{\mu \, Siron} = \dfrac{52.6.10^{-3}.2}{1.375 \; 10^{-2}.0.95.10^{-4}} = 8 . 10^4 \text{ A x turns / web}$

If we suppose an air gap equal to 0.10 mm and $\mu = r \, 10,000$ well'get:

$$R = {}_{air} 8.10^4 . \frac{0.10.10^4}{2 \; 52. \, 6} = 7.65 .10^5$$

Therefore

$$L = \frac{N^2}{R_a + R_{iron}} = \frac{845^2}{8 . 10^4 + 7.65 . 10^5} = 844 \text{ mH}$$

If the reactance, due to this inductance, is calculated, we'll get:

$$x = 2 \, \pi f . L = 2 \, \pi \, 6. \, 0.844 = 31.8 \, \Omega$$

from which we get a current:

$$I = \frac{V}{x_L} = \frac{10.7}{31.8} = 336 \text{ mA},$$

reasonable value.

The missing flux, associated to this magnetic field, will be such as to produce an induction equal to about 10 gauss at the one metre's distance from the device.

### 2.3.3.5. BABY CRY ALERT CIRCUIT

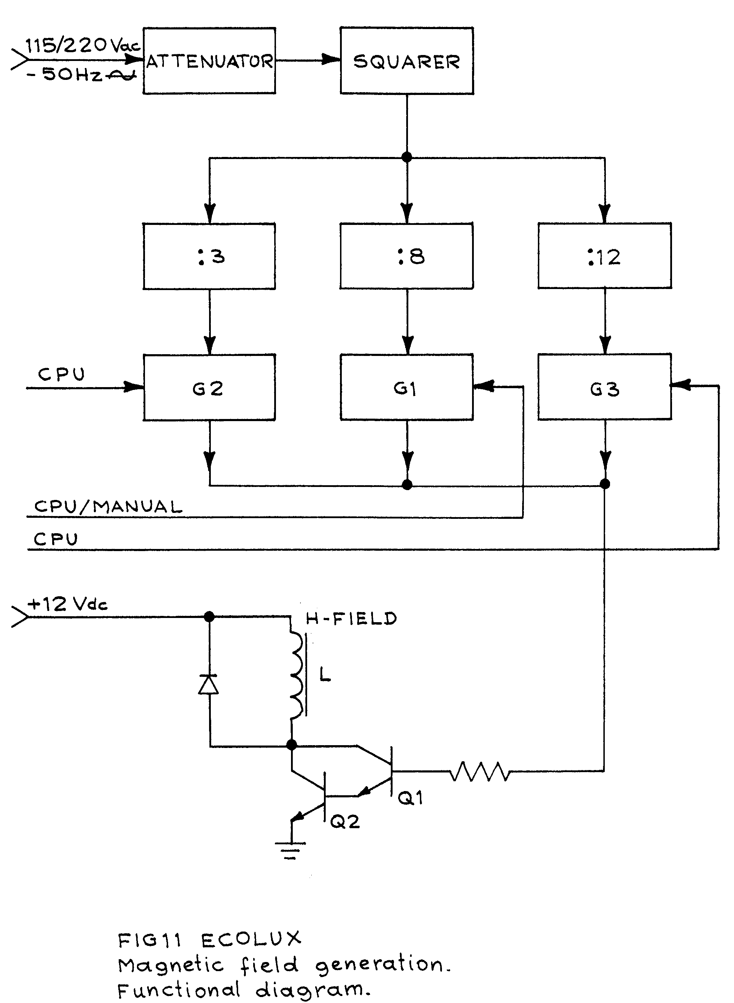

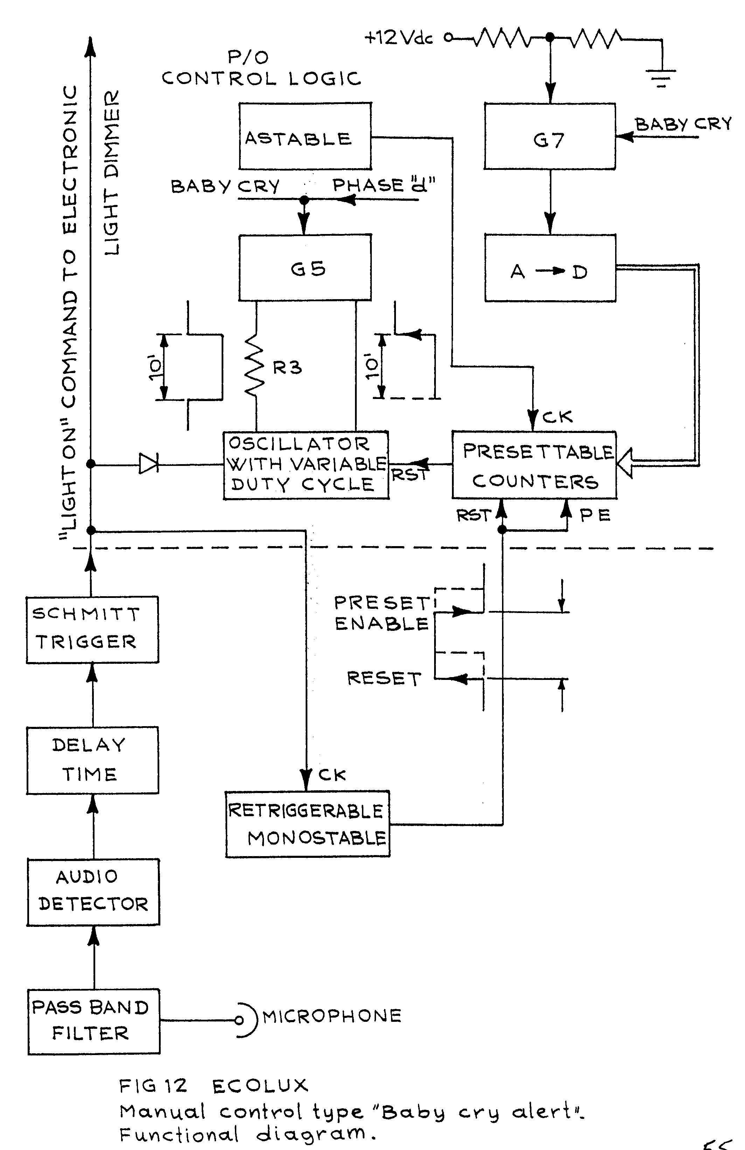

In fig. 12 functional diagram of "BABY CRY ALERT" is shown.

From effected experiments, frequency pattern relative to baby cry, has been deduced.

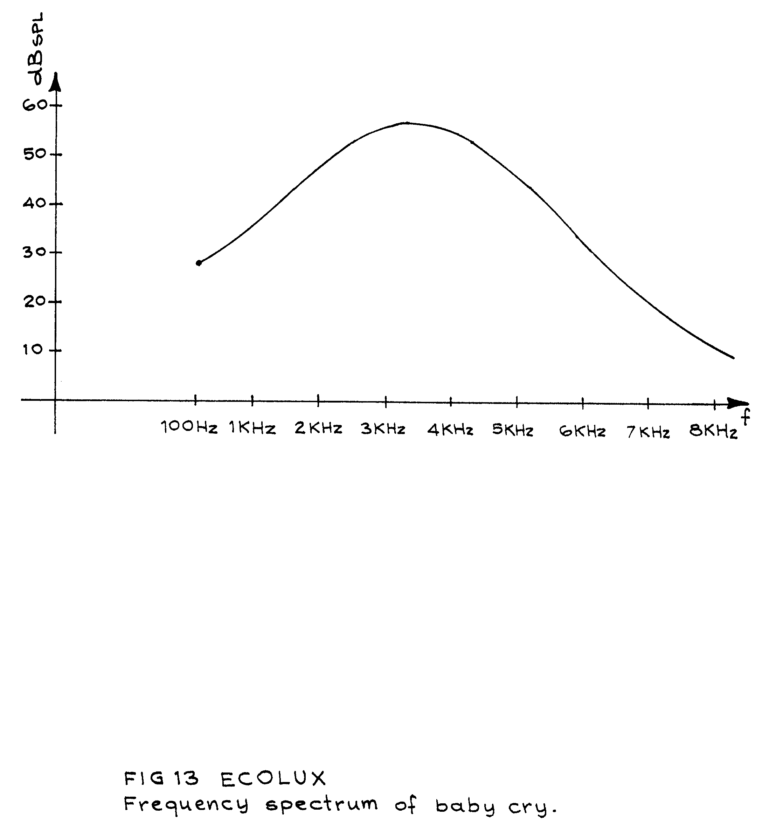

In fig. 13 such pattern is shown in order to determine the wave energy. Ordinate values correspond to sound pressure, and the abscissa values to frequency.

At it may be looked at, the energy is gathered mostly in frequency spectrum between 1500 hz and 5000 Hz, with a peak value placed about 2800/3000 Hz.

Therefore, in order to get a signal proportional to baby cry, it will be enough, by means of an active passband filter, to privilege such frequencies.

Besides these frequencies are applied to the successive audio detector.

After some seconds (time constant of delay time circuit), the light turns on and the impulse is applied to the monostable to produce a signal which resets and presets the Control Logic presettable counters.

A continous voltage, switched at baby cry by means of G7, after the analogic to digital conversion, provides to preset the counter for a counter time equal to ten minutes. This time must be considered since the baby stops crying, because the PRESET ENABLE signal is provided by a retrigerable monostable.

The time duration of passage from light "on" to "off" is determined by astable circuit having a variable duty cycle and a time constant equal to six minutes.

When "baby cry" circuit is activated, a pleasant and low music, which, together to light presence, helps to keep the child quiet and to contribute to its sleeping again.

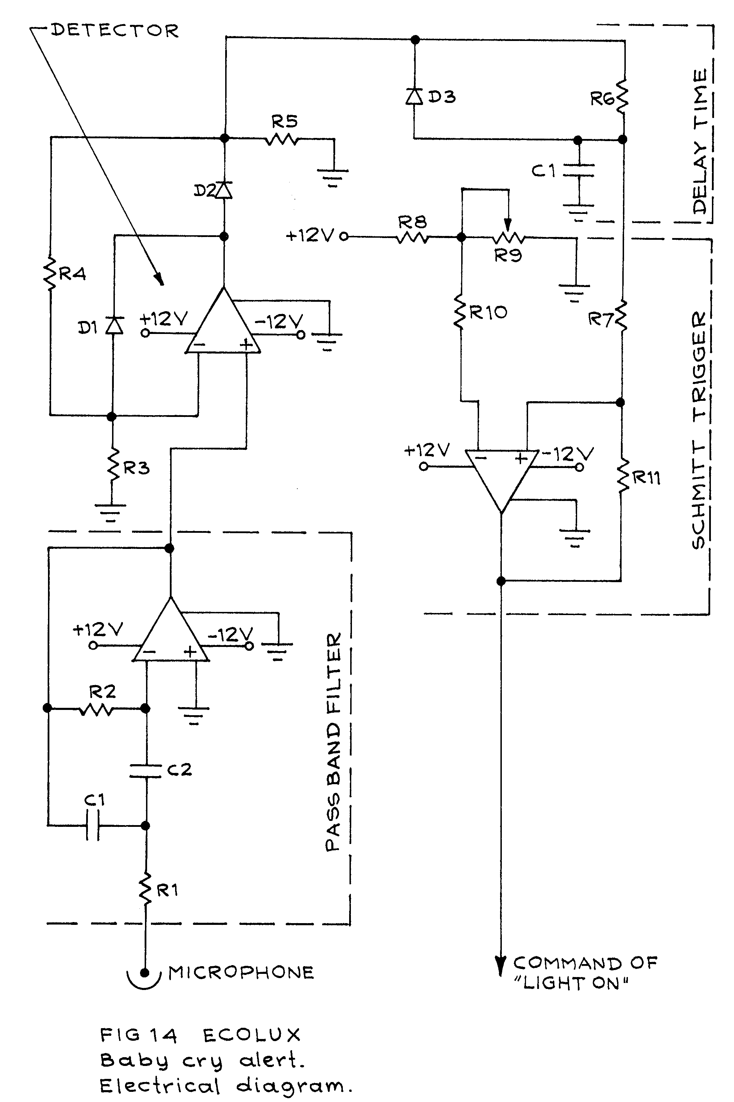

The fig.14 represents the electrical diagram of the "BABY CRY ALERT"

The passband filter having bandwidth from 1500 Hz to 5000Hz, is realized by one operational amplifier.

Another operational amplifier allows to detect the output audio signal: the detected voltage is present at C1, which together to R6, constitutes a delay time circuit of some seconds.

The output of the detector is applied to the trigger, which is used for enabling the following monostable.

### 2.3.3.6. POWER SUPPLY CIRCUIT

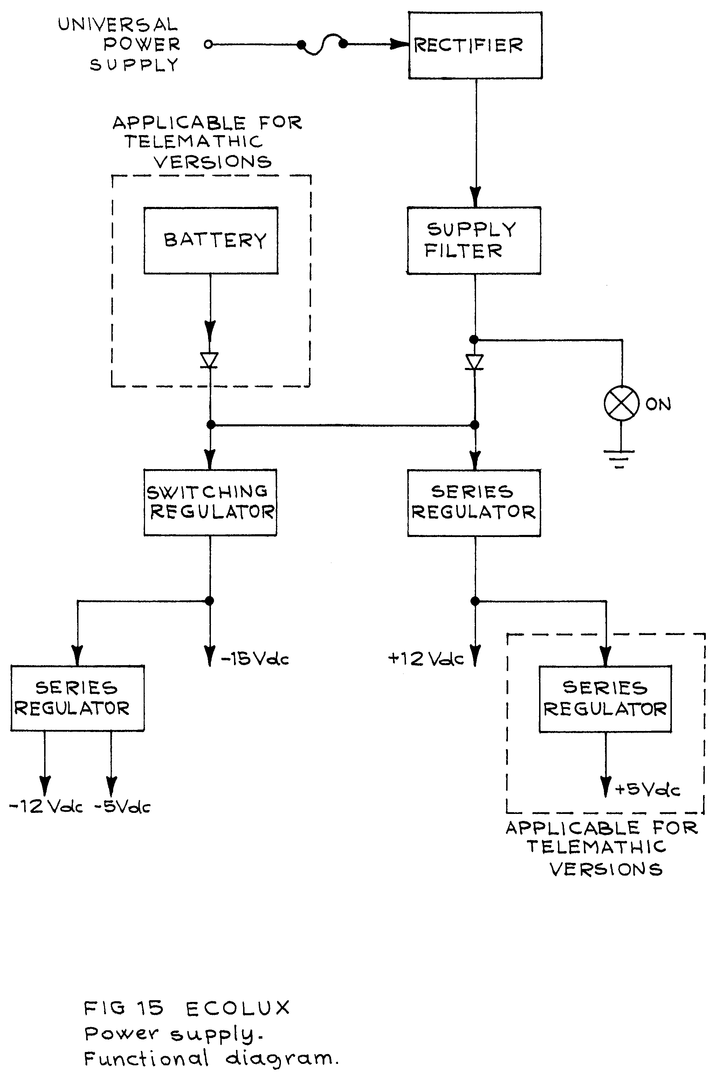

Power supply functional diagram of ECOLUX devices is shown in fig. 15.

The main voltage is rectified, filtered and applied to some regulatos, to get the necessary voltages.

By means of a series regulator, is got the + 12 Vdc voltage, necessary for L inductance, for operational circuits, and to obtain, by means of another series regulator, the + 5 Vdc voltage.

By means of a switching regulator, a - 15 Vdc voltage is obtained, necessary for electronic dimmer integrated circuit, and to get, by means of two series regulators, the dc voltages of -12 V and- 5 V.

A battery back - up is foreseen as an option for telemathic model to keep in store the program data, even if main voltage is absent.

### 2.3.4. SHORT FUNCTIONAL DESCRIPTION OF THE DIGITAL COMMAND MODEL WITHOUT CPU.

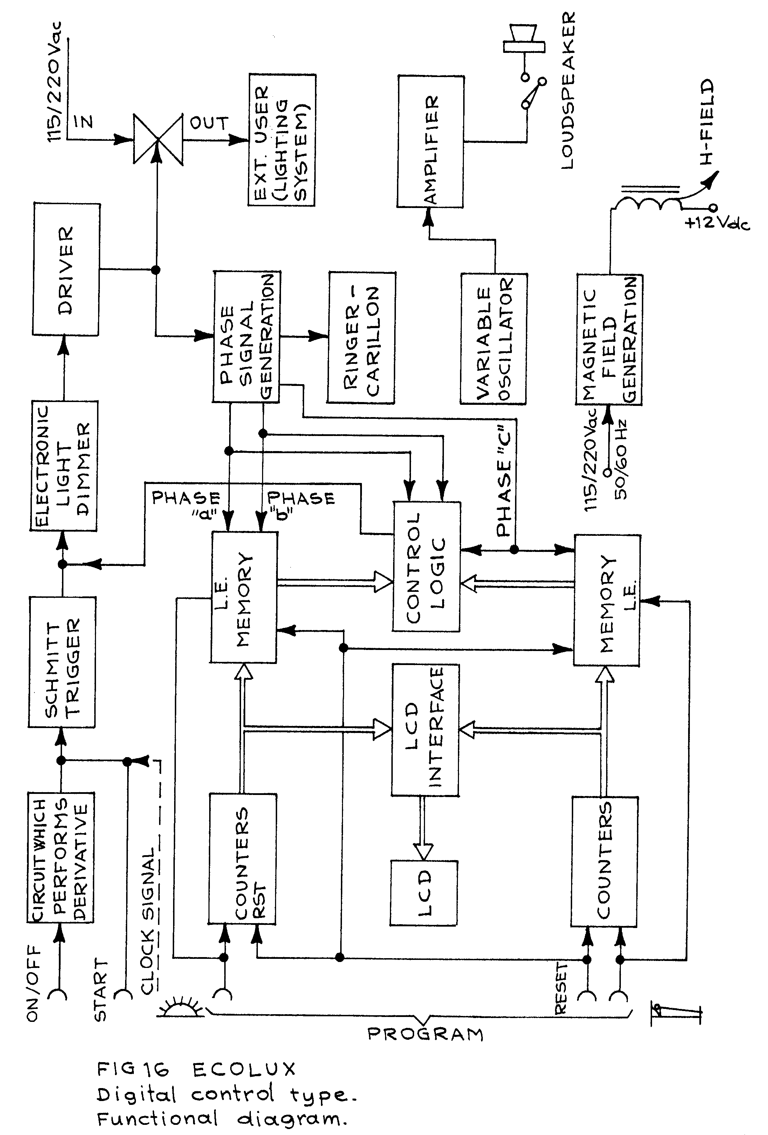

The functional diagram of this device is shown in fig. 16: by means of programming pushbuttons, the counter's counting is changed.

The state of the counters is then applied to LCD and is stored.

The stored signals are applied also to the control Logic, in order to get the light's desidered adjustement.

The operation of the other circuits is similar to that described for the manual command type.

The RESET pushbutton provides for resetting the counting for a new programming.

The control Logic circuit hasn't the analog to digital conversion, because the data which are proportional to necessary times to pass from light -"off" to "on" and from "on" to "off" and which get out from the memories, are already in digital form.

### 2.3.5.SHORT FUNCTIONAL DESCRIPTION OF CPU MODEL DEVICE.

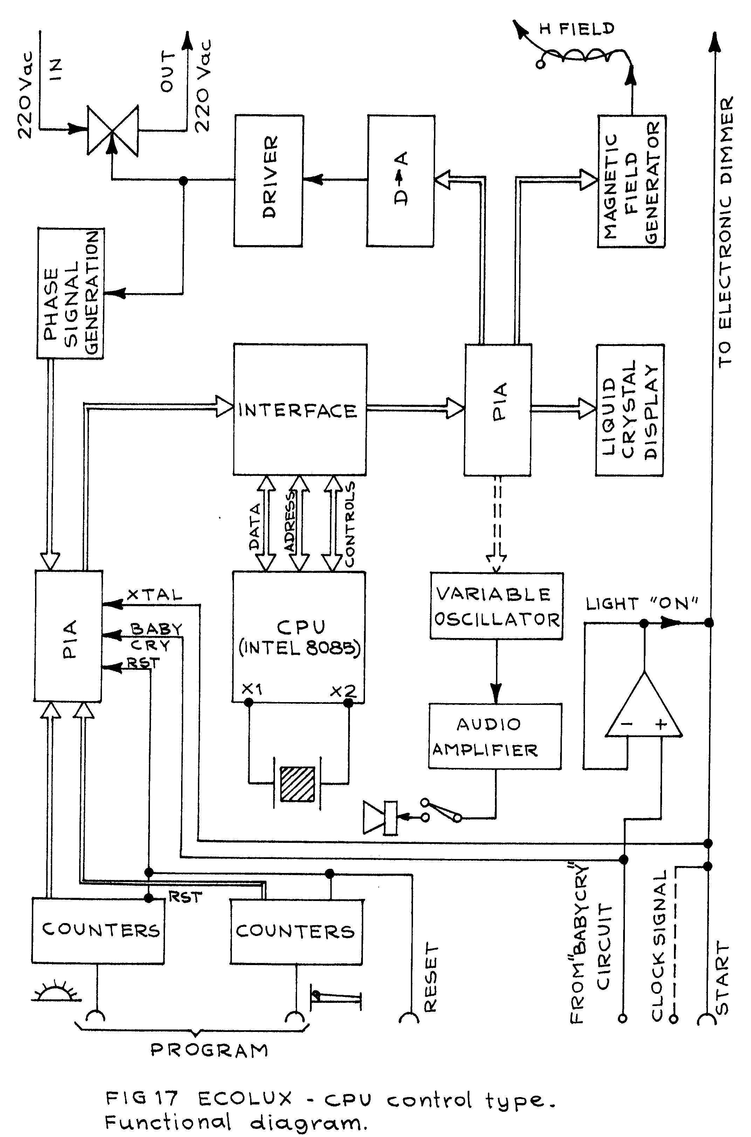

In fig. 17 the functional diagram of the CPU model is shown.

The utilized microprocessor is the 8 bit INTEL 8085.The program data, loaded into the counters, are sent through PIA to CPU which, by means of suitable interfaces, applies the processed data:

I) To liquid crystal display, where the set time parameters are read;

2) To digital to analogic converter, which by means of a driver acts on dimmer circuit to get the desired luminosity variation.

3) To magnetic field generator, to match the magnetic field frequencies to those generated by electrical cerebral waves and detected in the specific "brain wave diagram";

4) Possibly, to audio generator, in order to change musical tones and / or to start music to have a soporiphic effect (device custom direction).

2.3.6.ELECTRICAL AND MECHANICAL CHARACTERISTICS.

l) **Control parameters from light "on" to "off":**

ten different curves may be selected.
**The times of each phase are according to the following:**

Phase a) Duration time of light "on:

**it is variable from l′ to 20′.**

Phase b) Duration time for the light to pass from light "on" to light "off":

**it is variable from l′ to 30′.**

2)Control parameters from light "off" to light "on":

**Phase c):Period duration during which the light has a low luminous intensity ( light "off" = sleeping duration):**

it is variable from 30′ to twelve hours.

**Phase d) Necessary time to pass from light"off" to light "on":**

constant and equal to six minutes.

**3) Times of "plug - in" Baby Cry Alert:**

**a) Necessary time to pass from light "off" to light "on":**

after some seconds since baby has begun to cry.

**b) Period duration during which it is present the full luminous intensity (diffused lighting):**

ten minutes after since the baby has stopped crying.

**c) Period duration to pass from light "on" to "off":**

six minutes.

**4) Magnetic field generation**

a magnetic field of about ten gausses at rough one metre of distance from the device and of frequency equal to 6 Hz.

**5) CPU model characteristics:**

variable luminosity curves; variable frequencies of magnetic field and equal to 4Hz, 6 Hz and 16 Hz: such parameters are stored in the CPU according to the detected brain wave diagrams (custom program). Eventual management of the music by means of CPU.

**6) Outputs / inputs.**

N. I standard plug
N. I socket for 25 W to 150 W lamps having great base
N. I socket of universal type, adaptable to any plug (Vimar).

**7) Accessories:**

Extension cable having suitable Plug for connection to the wall.
Reducer for lamps having mignon base.
Reducer for other lamps.
Reducer for different wall socket.

**N. 2 types:**

a)On wall embedment type
b) For bedside installation and / or plugged portable type.

**g) Power supply:**

220 Vac / 115 Vac 50 - 60 Hz

**Power comsumption:**

less than 500 mw
A NO BREAK is available for telemathic version.

ECOLUX

APPLICATIONS/TYPES

APPLICATIONS                             USE/AIM

- HOSPITALS

- NURSING HOME                    MEDICAL / SANITARY

- THERAPEUTIC INSTITUTES

- CIVIL HOUSES

- HOTELS                              AS CONSUMER GOODS

PRODUCT TYPES
A - MANUAL CONTROL                   WITH UNIVERSAL SLOT
        (ECOLUX)             PORTABLE WITH CONNECTING PLUG
                             BEDSIDE TABLE INSTALLATION

B - AUTOMATIC CONTROL                WITH UNIVERSAL SLOT
    ( ECOLUX / TERALUX)      PORTABLE WITH CONNECTING PLUG
                             BEDSIDE TABLE INSTALLATION
                                          .

C - AUTOMATIC CONTROL                WITH UNIVERSAL SLOT
    (  WITH  CPU)            PORTABLE  WITH  CONNECTING PLUG
    ( TERALUX)               BEDSIDE TABLE INSTALLATION
                                          .

TABLE 1

EP 0 469 227 A1

ECOLUX

MAIN CHARACTERISTICS

UNIVERSAL POWER SUPPLY

NO BREAK

ADAPTABLE TO ANY PLUG/SOCKET AND/OR LAMP BASE

UP TO 500 W LOAD

ALARM RINGER

PLUG - IN FOR BABY CRY

SLIDER FOR SLEEPING LENGTH

LIGHTING - OFF STRAIGHT LINED CURVE ( N POSSIBILITIES)

SLEEP'S BIOLOGICAL CYCLE REGULATOR

AUTOMATIC START FOR EVERY 24 HOURS DUE TO PLUG - IN CLOCK

TOUCH CONTROL ON/OFF SWITCH

EVERY CYCLE'S DURATION ADJUSTMENT

CUSTOM CPU PROGRAM

SELECTION OF CYCLE'S NUMBER (UP TO 8)

POSSIBILITY OF A SWEET AND LOW SOUND

TRANSITION FROM ON TO OFF SIMILAR TO A "SUNSET"

GENERATION OF SLOWLY VARIABLE MAGNETIC FIELD

TABLE II

## Claims

1) Disposition according to which ECOLUX generates three parameters: light, sound and magnetic field, having the ability to control respective levels, in order to favour sleeping and to attenuate sleep's troubles of adults and/ or to make the crying during children'sleep stop,giving them quietness, by means of a suitable "plug - in" circuit; disposition according to which a "plug - in " clock is foreseen, in order to allow an automatic programming of sleeping lenght during the 24 hours of every day, by means of devices realized in two main types for on wall embedment installation or for bedside installation; disposition according to which such requirements are realized with three models: one having manual commands and two having digital commands (without or with CPU).

13

EP 0 469 227 A1

Any main voltages, any lamp, any plug, any socket may be applied to every portable type.

**2)** Disposition according to the claim I), by which a curve of the sheaf of automatic lighting - off curves (about ten), having a law similar to a sunset, may be selected.

Disposition according to the claim 1), by which the period, of sunset characteristic, is followed by a dim luminosity period, having variable intensity in accordance with sleeping stages, having duration selectable from 30 minutes to twelve hours.

**3)** disposition according to the claim I), by which a low level music is generated, by which this music has variable frequencies from 300 Hz to 10,000Hz, with a sweet and pleasant sound.

**4)** Disposition in accordance with the claim I), by which a magnetic field having about 10 gauss intensity at distance of one metre, having a fixed frequency of 6 Hz or a variable frequency of, about 4 Hz, 6 Hz, 16 Hz is generated, by which its management is done by CPU according to the sleeping stages.

**5)** Disposition in accordance with the claim I), by which the luminosity variation is done by means of an electronic dimmer integrated circuit, of relative transistor driver and of triac.

**6)** Disposition according the claim 5) by which the electronic dimmer is driven by the circuits necessary to light's manual adjustement by means of a touch control pushbutton (fig.7) and by which it may be driven besides by the circuit constituted essentially by an oscillator having a variable duty cycle, enabled by counting end of presettable counters.

**7)**Disposition, according to the claim 5) by which the command signal for the triac is used for getting (see fig.10), by means of two comparators and one flip - flop,the phase c signal (light - off) and the phase a signal (light - on) and, by means of a differentiator circuit, two comparators and one flip - flop, the phase b (light transition from "on" to "off") and the phase d (light transition from "off" to "on") signals.

**8)** Disposition according to the claim 5) by which a signal to enable a ringer at the middle of the lighting - on period is generated ; by which this signal is realized by the command voltage of the triac, applied to one window comparator, enabled during the phase d.

**9)** Disposition according to the claim I), by which a magnetic field is generated, by application of main 50/60 Hz voltage, suitably divided by 8 (without CPU) or by 3, by 8 and by 12 to an amplifier realized by darlington transistors, of which the collectors drive a C - core inductance (fig. II).

**10)** Disposition according to the claim I), by which a sensitive circuit to baby cries is obtained, by means of a passband filter which privileges the frequencies between I,500 Hz and 5000 Hz, peculiar frequency of the baby cry pattern, and by means of the subsequent audio detector. (see fig.12).

**II)** Disposition according to the claim 10), by which the passband filter is active and is realized by operational amplifier; by which the audio detector also is realized by means of an operational amplifier; by which the audio detector's output is applied to a delay time circuit, made by a resistor with a diode in parallel having its anode connected to the capacitor, such as to form a time constant of some seconds (see fig. 14).

**12)** Disposition according to claim II), by which the output signal from the delay circuit is sent to a Schmitt Trigger to generate the lighting - on signal and then to a retriggerable monostable, in order to generate the preset last pulse for the counters when crying stops.

**13)** Disposition according to the claim 12), by which the output signal from the monostable enables the preset counters, which fix in ten minutes the light on duration since baby stops crying and by which the output of counters enables the oscillator with variable duty cycle, which causes the duration of transition from light "on" to "off" be six minutes.

**14)** Disposition according to the claim 6), by which the counters are preset by codes coming from analogic to digital converter, in order to determine the counting period proportional to time duration of light "on" (phase a) or of a light "off" (phase c).

**15)** Disposition according to the claim 14), which the presettable counters are driven as clock signal by a low frequency oscillator during the phase c and by a high frequency oscillator during the phase a.

**16)** Disposition according to the claim I), by which the models having digital commands are realized with liquid crystal display.

**17)** Disposition according to the claim I), by which the CPU version uses an 8 bit microprocessor (INTEL 8085A), which provides for the management of variable luminosity curves, for the management of the magnetic field frequencies and for the music's eventual management,.

**18)** Disposition according to the claim 17), by which the microprocessor can maintain in memory the data relative to a specific "brain wave diagram"and allows besides to drive the triac with an analogic voltage derived by CPU digital codes converted into analogic signal by means of a suitable digital to analogic conversion.

**19)** Disposition according to the claim I), by which in the two digital command types, either with CPU or without CPU, a short NO BREAK is realized with a +5Vdc battery back - up to maintain in memory the program data.

14

FIG 1  ECOLUX
Typical cycle which may be started in dependance
of the detected " brain wave diagram" (such
cycle may be automatically repeated every 24 hours).

Fig.2 List of possible reasons capable to change the personal biological clock

a) WALL SOCKET INSTALLATION

COMMANDS/
CONTROLS

b) BEDSIDE INSTALLATION

FIG 3 ECOLUX
Possibility for wall (case a) and for
bedside installation (case b).

17

FIG 4 ECOLUX
Manual /automatic control capability.
Functional diagram.

FIG. 5 – VERSIONE A COMANDI MANUALI ECOLUX

FIG5 ECOLUX
MANUAL CONTROL TYPE
FRONT VIEW

EP 0 469 227 A1

TIME

① ② ③ ⑩ ⑧

⑦

ON/OFF   RESET   PROGRAM

ON

OFF

START

BABY CRY

⑤ ④ ⑥ ⑪ ⑨

FIG.6 - VERSIONE A COMANDI
DIGITALI ECOLUX

FIG 6   ECOLUX
DIGITAL CONTROL TYPE
FRONT VIEW

FIG 7 ECOLUX
Manual control type.
Functional diagram.

21

a) TRANSITION FROM "LIGHT ON" TO "LIGHT OFF"

b) TRANSITION FROM "LIGHT OFF" TO "LIGHT ON"
(SLEEPING LENGTH)

FIG 8 ECOLUX
Intervention times of luminosity
control device.

FIG 9 ECOLUX - Manual control type control logic - Functional diagram.
(For detailed electrical diagram refer to fig 9/1 and fig 9/2)

FIG 9/1 ECOLUX
Electronic light dimmer.
Electrical diagram.

FIG 9/2  ECOLUX
Manual control type time generator.
Electrical diagram.

FIG 10  ECOLUX
Manual control type
phase signal generation.
Functional diagram.

FIG11 ECOLUX
Magnetic field generation.
Functional diagram.

FIG 12   ECOLUX
Manual control type "Baby cry alert".
Functional diagram.

28

FIG 13 ECOLUX
Frequency spectrum of baby cry.

FIG 14 ECOLUX
Baby cry alert.
Electrical diagram.

FIG 15 ECOLUX
Power supply.
Functional diagram.

31

FIG 16 ECOLUX
Digital control type.
Functional diagram.

FIG 17 ECOLUX - CPU control type.
Functional diagram.

**European Patent**
**Office**

**EUROPEAN SEARCH REPORT**

Application Number

EP 90 83 0570

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-U-8 904 492 (GOLF GmbH) <br> * Page 3, lines 12-32; page 4, line 16 - page 5, line 4; page 5, line 31 - page 6, line 26; in particular page 6, lines 8-19 * | 1 | A 61 M 21/00 |
| A | FR-A-2 645 659 (REGNARD) <br> * Abstract; page 5, lines 14-19; figure 1 * | 1-3 | |
| A | WO-A-8 500 264 (CAPEL) <br> * The whole document, in particular page 4, lines 11-15 * | 5 | |
| A | WO-A-8 810 091 (BRIGHAM et al.) <br> * Abstract; claims 1,2; figure 1 * | 1 | |
| A | EP-A-0 058 564 (PEARLING) <br> * Page 8, lines 8-13 * | 4 | |
| A | DE-A-3 838 351 (LERCHL) <br> * Abstract * | 10 | TECHNICAL FIELDS SEARCHED (Int. Cl.5) |
| | | | A 61 M <br> A 61 N |

The present search report has been drawn up for all claims

| Place of search | Date of completion of the search | Examiner |
|---|---|---|
| THE HAGUE | 23-10-1991 | PAPONE F. |

EPO FORM 1503 03.82 (P0401)